# EUROPEAN PATENT SPECIFICATION

(11) **EP 1 895 935 B1**
(45) Date of publication and mention of the grant of the patent: **16.04.2014**
(21) Application number: 06784980.2
(22) Date of filing: 15.06.2006
(51) Int. Cl.: A61F 2/01

(54) **INTRAVASULAR FILTER**
ENDOVASKULÄRER FILTER
FILTRE INTRAVASCULAIRE

(30) Priority: 20.06.2005 US 156426
(43) Date of publication of application: 12.03.2008
(73) Proprietor: Boston Scientific Limited, St. Michael, Barbados, West Indies (BB)
(72) Inventor: RENATI, Richard, J., Los Gatos, California 95032 (US)
(74) Representative: Frhr.Schenck zu Schweinsberg, Elard
(86) International application number: PCT/US2006/023454
(87) International publication number: WO 2007/001902

(56) References cited:
- WO-A-98/39053
- WO-A-98/51237
- US-A- 6 129 739

## Description

The invention relates generally to intravascular filters and methods of their formation. In particular, the invention relates to intravascular filters configured for improved vasculature interaction.

Heart and vascular disease are major problems in the United States and throughout the world. Conditions such as atherosclerosis result in blood vessels becoming blocked or narrowed. This blockage can result in lack of oxygenation of the heart, which has significant consequences since the heart muscle must be well oxygenated in order to maintain its blood pumping action.

Occluded, stenotic, or narrowed blood vessels may be treated with a number of relatively non-invasive medical procedures including percutaneous transluminal angioplasty (PTA), percutaneous transluminal coronary angioplasty (PTCA), and atherectomy. Angioplasty techniques typically involve the use of a balloon catheter. The balloon catheter is advanced over a guidewire such that the balloon is positioned adjacent a stenotic lesion. The balloon is then inflated and the restriction of the vessel is opened. During an atherectomy procedure, the stenotic lesion may be mechanically cut away from the blood vessel wall using an atherectomy catheter.

During angioplasty and atherectomy procedures, embolic debris can be separated from the wall of the blood vessel. If this debris enters the circulatory system, it could block other vascular regions including the neural and pulmonary vasculature. During angioplasty procedures, stenotic debris may also break loose due to manipulation of the blood vessel.

Because of this debris, a number of devices, such as intravascular filters, have been developed to filter out this debris.

For example, US 6,129,739 discloses apparatus and methods for use in filtering emboli from a vessel and performing thrombectomy and embolectomy, wherein a vascular device comprises one or more support hoops, each having an articulation region connected near a distal end of a guide wire, and a blood permeable sac affixed to the support hoop or hoops to form a mouth of the blood permeable sac. The mouth of the sac closes when the apparatus is collapsed for removal to prevent material from escaping from the sac.

A need remains for improved intravascular filters. A need remains for improved methods of manufacture of intravascular filters.

The invention is directed to intravascular filters such as embolic protection filters according to claim 1 that are configured to capture embolic debris when deployed within a patient's vasculature, and to a method of forming a filter according to claim 12.

Accordingly, an embodiment of the invention can be found in an embolic protection filter that includes a support hoop, a filter membrane having a proximal region encapsulating the support hoop, and a filter fillet that is positioned proximate the support hoop and that extends proximally from the support hoop. The filter fillet is configured with a shape that provides for relatively smooth blood flow between a region upstream of the filter and an open month and of the filter.

The filter according to the present invention may be utilized in the following method of capturing stagnant emboli. According to said method a filter is provided, the filter comprising a support hoop and a filter membrane having a proximal portion. The filter membrane includes an extension portion extending proximally from the support hoop. The filter is deployed within a vasculature including vascular walls, such that the proximal portion of the filter membrane and the extension portion contact the vascular walls. Finally, the filter is removed. According to said method, the extension portion is configured to reduce stagnant flow proximate the support loop and vascular wall.

The invention may be more completely understood in consideration of the following detailed description of various embodiments of the invention in connection with the accompanying drawings, in which:
Figure 1 is a perspective view of an intravascular filter in accordance with an embodiment of the invention;
Figure 2 is a closer view of a portion of the filter membrane included in the intravascular filter of Figure 1;
Figure 3 is a perspective view of the intravascular filter of Figure 1, shown deployed within an artery or vein; and
Figure 4 is an axial cross-section view of a portion of Figure 3.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention.

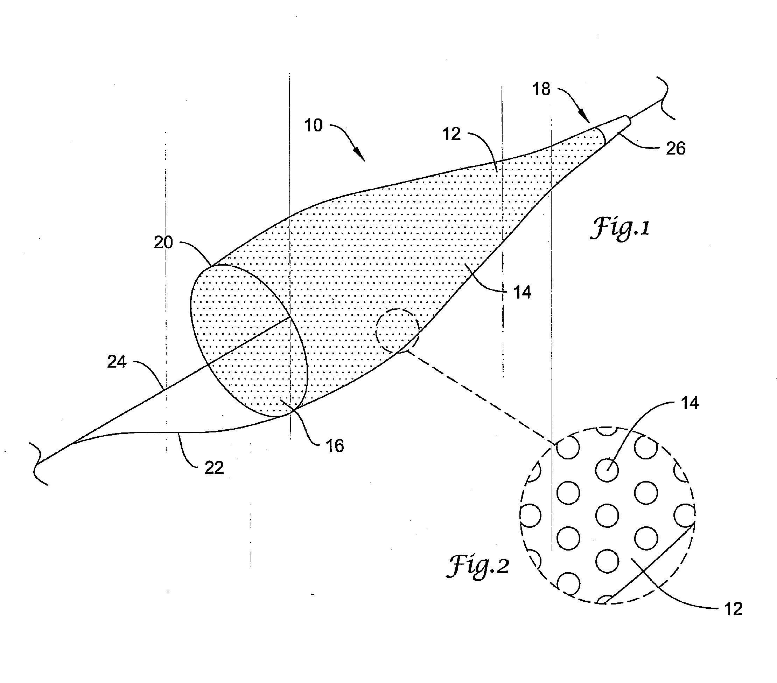

Figure 1 is a perspective view of an example intravascular filter 10, which includes a filter membrane 12. The filter membrane 12 can be formed from any suitable moldable material or combination of materials. For example, the filter membrane 12 can include polymers such as polyether block amide, polybutylene terephthalate/polybutylene oxide copolymers sold under the Hytrel and Arnitel trademarks, Nylon 11, Nylon 12, polyurethane, polyethylene terephthalate, polyvinyl chloride, polyethylene naphthalene dicarboxylate, olefin/ionomer copolymers, polybutylene terephthalate, polyethylene naphthalate, ethylene terephthalate, butylene terephthalate, ethylene naphthalate copolymers, polyamide/polyether/polyester polyamides, aromatic polyamides, polyurethanes, aromatic polyisocyanates, polyetheretherketone, polycarbonates, polyamide/polyether, and polyester/polyether block copolymers, among others.

In some embodiments, the filter membrane 12 can be formed from at least one of polyether block amide, olefin/ionomer copolymers, nylon, polyurethane, polyethylene terephthalate, polyvinyl chloride, polyethylene naphthalene -dicarboxylate and mixtures or copolymers thereof.

The filter membrane 12 can be porous, having pores 14 that are configured to permit blood flow while retaining embolic material of a desired size. The pores 14 are seen in greater detail in Figure 1. While the pores 14 as illustrated are at least substantially circular in profile, other profiles are contemplated as well. The filter membrane 12 can have a mouth 16 and a closed end 18 and is capable of moving between an open state and a closed state. The mouth 16 can be sized to occlude the lumen of the body vessel in which the filter may be installed, thereby directing all fluid and any emboli through the filter.

A support hoop 20 can be attached to the filter membrane 12 at or proximal to the mouth 16. The support hoop 20 can be attached to the filter membrane 12 through melt bonding or other suitable means. In some embodiments, as discussed in greater detail hereinafter, the support hoop 20 can be integrally molded within the filter membrane 12. The support hoop 20 has an expanded state and a compressed state. The expanded state of the support hoop 20 is configured to urge the mouth 16 to its full size, while the compressed state permits insertion into a small lumen.

The support hoop 20 can be made from a flexible metal such as spring steel, from a super-elastic elastic material such as a suitable nickel-titanium alloy, or from other suitable material. The support hoop 20 can be a closed hoop made from a wire of uniform diameter, it can be a closed hoop made from a wire having a portion with a smaller diameter, it can be an open hoop having a gap, or it can have another suitable configuration.

A strut 22 can be fixedly or slideably attached to and extend from the support hoop 20. An elongate member 24 can be attached to and extend from the strut 22. The elongate member 24 can be attached to the strut 22 at an angle or the strut 22 can have a small bend, either at a point or over a region. The strut 22 can be attached to the support hoop 20 at a slight angle such that when the elongate member 24, the strut 22, and the support hoop 20 are in an unconstrained position, the elongate member 24 can generally extend perpendicular to the support hoop 20.

In the unconstrained position, the elongate member 24 can also lie along an axis which passes through the center of the region created by the support hoop 20. This may help position the support hoop 20 in contact with the wall of a vascular lumen or it may help in enhancing predictability or reliability during deployment. In some embodiments, the elongate member 24 can terminate at the strut 22. In other embodiments, the elongate member 24 can extend through the filter membrane 12, as shown. Whether or not the elongate member 24 extends through the filter membrane 12, it may be fixedly or slideably/rotatably attached to the filter membrane 12.

The filter membrane 12 can include a waist 26 at the closed end 18. In some embodiments, the waist 26 can be integrally formed with the filter membrane 12. In other embodiments, the filter membrane 12 can be further processed to form the waist 26. In some embodiments, integrally forming the waist 26 with the filter membrane 12 can reduce the outer diameter of the filter -device when in a compressed state, increase the reliability and uniformity of the bond between the filter membrane and the elongate member, and reduce the number of steps or components needed to form the filter device.

The waist 26 is a region largely incapable of moving between two states and having a lumen of substantially constant diameter therethrough. The elongate member 24 can extend through and be bonded to the waist 26. This bonding can be heat bonding such as laser bonding or may be an adhesive or other suitable means.

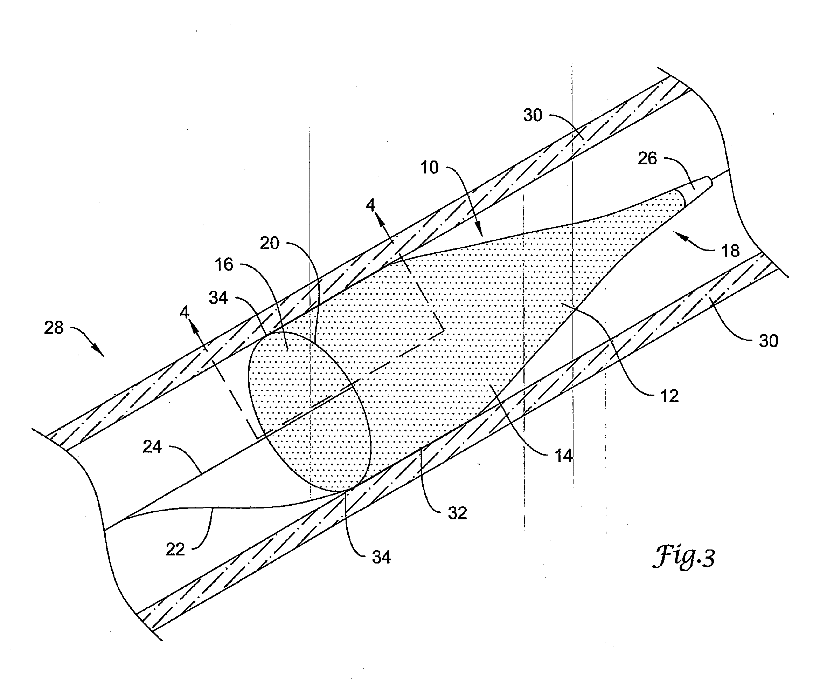

With respect to Figure 3, the intravascular filter 10 can be deployed within a vasculature 28 that can include an artery or a vein within a patient. For illustration purposes only, the vasculature 28 will be referred to herein as the vessel 28. In some embodiments, the vessel 28 can include vessel walls 30. As can be seen, the open mouth end 16 can be in substantial contact with the vessel walls 30 at a contact point 34. In some embodiments, at least a proximal portion 32 of the filter membrane 12 can also be in substantial contact with the vessel walls 30, depending on the particular geometry of the vessel 28.

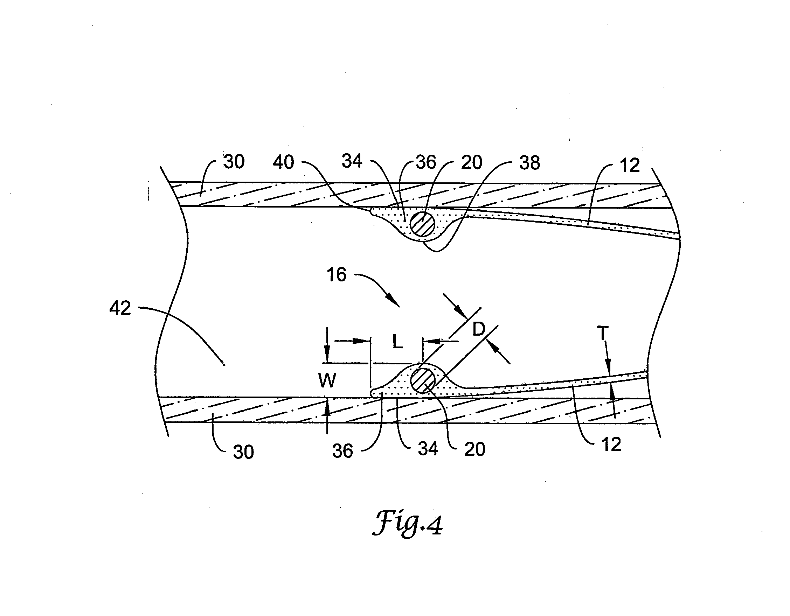

Figure 4 is an axially aligned partial section view of Figure 3, taken near the contact point 34. In this illustration, the support hoop 20 can be seen embedded within the filter membrane 12. A fillet 36 that is integrally formed with the filter membrane 12 extends proximally beyond the support hoop 20. The fillet 36 can extend circumferentially at least partially around the support hoop 20. In some embodiments, the fillet 36 can extend circumferentially substantially or completely around the support hoop 20.

As can be seen in Figure 4, the fillet 36 provides a more gradual transition to the open mouth end 16 of the intravascular filter 10. The fillet 36 can taper from a more distal position 38 proximate the support hoop 20 to a proximal position 40. At the distal position 38, the fillet 36 can have a material thickness T that is about equal to that of the filter membrane 12. The fillet 36 can have an overall thickness W at the distal position 38 that is about equal to a cross-sectional diameter D of the support hoop 20 plus twice the thickness T of the filter membrane 12.

At the proximal position 40, the fillet 36 can have a thickness W that tapers to about zero. The fillet 36 can have any suitable length L. In some embodiments, the fillet 36 has a length L that is about one to four times a cross-sectional diameter D of the support hoop 20. In particular embodiments, the fillet 36 can have a length L that is about one to two times the cross-sectional diameter D of the support hoop 20.

The intravascular filter 10 can be built to any suitable dimensions. In some embodiments, the support hoop 20 can have a cross-sectional diameter D that is in the range of about 0.001" to about 0.010" (0,00254 to about 0,0254 cm). In some embodiments, the filter membrane 12 can have a thickness T that is in the range of about 0.0004" to about 0.003" (0,00102 to about 0,00762 cm). In such embodiments, the fillet 36 can have an about overall thickness W that is in the range of about 0.002" to about 0.016" (0,00508 to about 0,0406 cm) (equal to D + 2T) and a length L that is in the range of about 0.001" to about 0.004" (0,00254 to about 0,0102 cm) (1D to 4D) and in particular embodiments a length L that is in the range of about 0.010" to about 0.040" (1D to 2D) (0,00254 to about 0,102 cm).

The fillet 36 can be configured with any suitable shape or profile, provided that the shape or profile provides for relatively smooth blood flow between a region 42 that is upstream of the intravascular filter 10 and the open mouth end 16 of the intravascular filter 10. In some embodiments, providing for relatively smooth blood flow in this region can reduce or eliminate the formation or collection of emboli that could otherwise form or collect just upstream of the contact point 34. As shown, the fillet 36 tapers linearly from the distal position 38 to the proximal position 40. In other embodiments, the fillet 36 can taper in a concave or convex configuration between the distal position 38 and the proximal portion 40.

The filter membrane 12 can be formed in any suitable manner. In some embodiments, the filter membrane 12 can be formed using spray molding in which an appropriately snapeu mandrel is provided. A number of layers of polymeric material are sprayed onto the mandrel to form the filter membrane. After several layers of polymeric material are sprayed onto the mandrel, the support loop 20 can be positioned over the mandrel, and additional polymeric layers can be sprayed onto the mandrel. In some embodiments, the fillet 36 can be formed by positioning the support loop 20 an appropriate distance distally of the proximal end of the sprayed polymeric layers. As a result, the support loop 20 is encapsulated within the filter membrane 12.

In some embodiments, the tapered profile of the fillet 36 can be created by masking during the spray molding process. In some embodiments, the tapered profile of the fillet 36 can be formed in a post-spraying grinding or milling process.

It should be understood that this disclosure is, in many respects, only illustrative. Changes may be made in details_{,} particularly in matters of shape, size, and arrangement of steps without exceeding the scope of the invention. The invention's scope is, of course, defined in the language in which the appended claims are expressed.

## Claims

1. An embolic protection filter (10) comprising:
a support hoop (20);
a filter membrane (12) having a distal region and a proximal region, the proximal region of the filter membrane encapsulating the support hoop (20); and
a filter fillet (36) positioned proximate the support hoop and extending proximally therefrom;
wherein the fillet (36) is configured with a shape that provides for relatively smooth blood flow between a region upstream of the filter (10) and an open mouth end (16) of the filter.

2. The filter of claim 1, wherein the filter fillet (36) comprises a material that is the same as that of the filter membrane (12).

3. The embolic protection filter of claim 1, further comprising a guidewire (24).

4. The embolic protection filter of claim 3, wherein the distal region of the filter membrane (12) is secured to the guidewire (24).

5. The embolic protection filter of claim 3, further comprising a strut (22) extending from the guidewire (24) to the support hoop (20).

6. The embolic protection filter of claim 1, wherein the filter fillet (36) comprises an integral extension of the filter membrane (12).

7. The embolic protection filter of claim 1, wherein the filter fillet (36) has a distal end and a proximal end, and the distal end of the filter fillet is positioned proximate the proximal region of the filter membrane.

8. The embolic protection filter of claim 7, wherein the filter fillet (36) has a distal thickness at its distal end and a proximal thickness at its proximal end, and the proximal thickness is less than the distal thickness.

9. The embolic protection filter of claim 7, wherein the filter fillet (36) tapers from a first thickness that is approximately equal to or greater than a diameter of the support hoop (20) to a second thickness that is approximately zero.

10. The embolic protection filter of claim 1, wherein the filter fillet (36) has a length defined between a proximal end of the filter fillet and the support hoop (20), where the length is about one to four times a cross-sectional diameter of the support hoop.

11. The embolic protection filter of claim 10, wherein the filter fillet length is about one to two times the cross-sectional diameter of the support hoop.

12. A method of forming a filter (10), comprising steps of:
providing a mandrel;
providing a support hoop (20);
forming a filter membrane (12) having a distal region and a proximal region by spraying a number of layers of polymeric material onto the mandrel, positioning the support hoop (20) over the mandrel, and spraying additional polymeric layers onto the mandrel;
wherein the support hoop (20) is positioned such that the proximal region of the filter membrane (12) forms around the support hoop (20), thereby encapsulating the support hoop;
wherein the filter membrane includes a fillet (36) integrally formed with the filter membrane (12) that extends proximally from the support hoop (20);
wherein the fillet (36) tapers from a distal position proximate the support hoop to a proximal position.

13. The method of claim 12, wherein the fillet (36) is shaped via masking during the spray molding process.

14. The method of claim 12, wherein the fillet (36) is shaped via a grinding or milling process subsequent to the spray molding process.

15. The method of claim 12, wherein the fillet (36) is shaped to reduce stagnant flow proximate the fillet when the filter is deployed.

## Patentansprüche

1. Embolieprotektionsfilter (10), das aufweist:
einen Stützreifen (20);
eine Filtermembran (12) mit einem distalen Bereich und einem proximalen Bereich,
wobei der proximale Bereich der Filtermembran den Stützreifen (20) verkapselt; und
eine Filterwulst (36), die nahe dem Stützreifen positioniert ist und sich proximal davon erstreckt;
wobei die Wulst (36) mit einer Form konfiguriert ist, die für relativ ungestörten Blutfluss zwischen einem Bereich stromaufwärts vom Filter (10) und einem offenen Mündungsende (16) des Filters sorgt.

2. Filter nach Anspruch 1, wobei die Filterwulst (36) ein Material aufweist, das mit dem der Filtermembran (12) identisch ist.

3. Embolieprotektionsfilter nach Anspruch 1, ferner mit einem Führungsdraht (24).

4. Embolieprotektionsfilter nach Anspruch 3, wobei der distale Bereich der Filtermembran (12) am Führungsdraht (24) befestigt ist.

5. Embolieprotektionsfilter nach Anspruch 3, ferner mit einer Strebe (22), die sich vom Führungsdraht (24) zum Stützreifen (20) erstreckt.

6. Embolieprotektionsfilter nach Anspruch 1, wobei die Filterwulst (36) eine integrale Erweiterung der Filtermembran (12) aufweist.

7. Embolieprotektionsfilter nach Anspruch 1, wobei die Filterwulst (36) ein distales Ende und ein proximales Ende hat und das distale Ende der Filterwulst nahe dem proximalen Bereich der Filtermembran positioniert ist.

8. Embolieprotektionsfilter nach Anspruch 7, wobei die Filterwulst (36) eine distale Dicke an ihrem distalen Ende und eine proximale Dicke an ihrem proximalen Ende hat und die proximale Dicke kleiner als die distale Dicke ist.

9. Embolieprotektionsfilter nach Anspruch 7, wobei sich die Filterwulst (36) von einer ersten Dicke, die etwa gleich groß oder größer als ein Durchmesser des Stützreifens (20) ist, auf eine zweite Dicke verjüngt, die etwa null ist.

10. Embolieprotektionsfilter nach Anspruch 1, wobei die Filterwulst (36) eine Länge hat, die zwischen einem proximalen Ende der Filterwulst und dem Stützreifen (20) definiert ist, wobei die Länge etwa das Ein- bis Vierfache eines Querschnittdurchmessers des Stützreifens beträgt.

11. Embolieprotektionsfilter nach Anspruch 10, wobei die Filterwulstlänge etwa das Ein- bis Zweifache des Querschnittdurchmessers des Stützreifens beträgt.

12. Verfahren zur Bildung eines Filters (10) mit den Schritten:
Bereitstellen eines Dorns;
Bereitstellen eines Stützreifens (20);
Bilden einer Filtermembran (12) mit einem distalen Bereich und einem proximalen Bereich durch Spritzen einer Anzahl von Schichten aus Polymermaterial auf den Dorn, Positionieren des Stützreifens (20) über dem Dorn und Spritzen zusätzlicher Polymerschichten auf den Dorn;
wobei der Stützreifen (20) so positioniert wird, dass sich der proximale Bereich der Filtermembran (12) um den Stützreifen (20) bildet, wodurch er den Stützreifen verkapselt;
wobei die Filtermembran eine mit der Filtermembran (12) integral ausgebildete Wulst (36) aufweist, die sich vom Stützreifen (20) proximal erstreckt;
wobei sich die Wulst (36) von einer distalen Position nahe dem Stützreifen zu einer proximalen Position verjüngt.

13. Verfahren nach Anspruch 12, wobei die Wulst (36) über Maskieren während des Spritzformgebungsvorgangs geformt wird.

14. Verfahren nach Anspruch 12, wobei die Wulst (36) über einen Schleif- oder Fräsvorgang im Anschluss an den Spritzformgebungsvorgang geformt wird.

15. Verfahren nach Anspruch 12, wobei die Wulst (36) so geformt wird, dass stagnierender Durchfluss nahe der Wulst reduziert ist, wenn das Filter gesetzt ist.

## Revendications

1. Filtre de protection embolique (10), comprenant :
un cerceau de support (20) ;
une membrane de filtre (12) présentant une zone distale et une zone proximale, la zone proximale de la membrane de filtre entourant le cerceau de support (20) ; et
un filet de filtre (36) disposé à proximité du cerceau de support (20) et s'étendant proximalement à partir de celui-ci ;
le filet (36) étant prévu avec une forme permettant d'obtenir un écoulement sanguin relativement égal entre une région en amont du filtre (10) et une extrémité d'embouchure ouverte (16) du filtre.

2. Filtre selon la revendication 1, où le filet de filtre (36) est constitué d'un matériau identique à celui de la membrane de filtre (12).

3. Filtre de protection embolique selon la revendication 1, comprenant en outre un fil-guide (24).

4. Filtre de protection embolique selon la revendication 3, où la zone distale de la membrane de filtre (12) est fixée au fil-guide (24).

5. Filtre de protection embolique selon la revendication 3, comprenant en outre une traverse (22) s'étendant du fil-guide (24) au cerceau de support (20).

6. Filtre de protection embolique selon la revendication 1, où le filet de filtre (36) comprend une extension intégrée de la membrane de filtre (12).

7. Filtre de protection embolique selon la revendication 1, où le filet de filtre (36) a une extrémité distale et une extrémité proximale, et où l'extrémité distale du filet de filtre est située à proximité de la zone proximale de la membrane de filtre.

8. Filtre de protection embolique selon la revendication 7, où le filet de filtre (36) présente une épaisseur distale à son extrémité distale et une épaisseur proximale à son extrémité proximale, et où l'épaisseur proximale est inférieure à l'épaisseur distale.

9. Filtre de protection embolique selon la revendication 7, où le filet de filtre (36) s'effile d'une première épaisseur sensiblement égale ou supérieure à un diamètre du cerceau de support (20) vers une deuxième épaisseur sensiblement nulle.

10. Filtre de protection embolique selon la revendication 1, où le filet de filtre (36) présente une longueur définie entre une extrémité proximale du filet de filtre et le cerceau de support (20), ladite longueur étant sensiblement égale à une à quatre fois un diamètre de section transversale du cerceau de support.

11. Filtre de protection embolique selon la revendication 10, où la longueur du filet de filtre est sensiblement égale à une à deux fois le diamètre de section transversale du cerceau de support.

12. Procédé de formation d'un filtre (10), comprenant les étapes suivantes :
préparation d'un mandrin ;
préparation d'un cerceau de support (20) ;
formation d'une membrane de filtre (12) présentant une zone distale et une zone proximale par pulvérisation d'un certain nombre de couches de matériau polymère sur le mandrin, mise en place du cerceau de support (20) sur le mandrin, et pulvérisation de couches polymères additionnelles sur le mandrin ;
le cerceau de support (20) étant mis en place de telle manière que la zone proximale de la membrane de filtre (12) soit formée autour du cerceau de support (20), entourant ainsi le cerceau de support (20) ;
la membrane de filtre comportant un filet (36) formé d'un seul tenant avec la membrane de filtre (12) et qui s'étend proximalement à partir du cerceau de support (20) ;
le filet de filtre (36) s'effilant d'un emplacement distal à proximité du cerceau de support vers un emplacement proximal.

13. Procédé selon la revendication 12, où le filet (36) est formé par masquage pendant le processus de moulage par pulvérisation.

14. Procédé selon la revendication 12, où le filet (36) est formé par processus de broyage ou mouture consécutif au processus de moulage par pulvérisation.

15. Procédé selon la revendication 12, où le filet (36) est formé pour réduire la stagnation de flux à proximité du filet quand le filtre est déployé.
